(19) Europäisches Patentamt

European Patent Office

Office européen des brevets

(11) **EP 0 897 693 B1**

(12) **EUROPÄISCHE PATENTSCHRIFT**

(45) Veröffentlichungstag und Bekanntmachung des Hinweises auf die Patenterteilung:
**10.11.2004 Patentblatt 2004/46**

(21) Anmeldenummer: **98202759.1**

(22) Anmeldetag: **18.08.1998**

(51) Int Cl.⁷: **A61B 5/06**, A61M 25/01, H02N 15/00

(54) **Verfahren zur Navigation eines magnetischen Objektes und MR-Anordnung**

Method for navigating a magnetic object and Magnetic-Resonance-Apparatus

Procedé de navigation d'un objet magnetique et dispositif de resonance magnetique

(84) Benannte Vertragsstaaten:
**DE FR GB**

(30) Priorität: **20.08.1997 DE 19736030**

(43) Veröffentlichungstag der Anmeldung:
**24.02.1999 Patentblatt 1999/08**

(73) Patentinhaber:
• **Philips Intellectual Property & Standards GmbH**
**20099 Hamburg (DE)**
• **Koninklijke Philips Electronics N.V.**
**5621 BA Eindhoven (NL)**

(72) Erfinder:
• **Kuhn, M Dr., c/o Philips Patentverwaltung GmbH**
**22335 Hamburg (DE)**

• **Aldefeld,B.,Dr., Philips Patentverwaltung GmbH**
**22335 Hamburg (DE)**

(74) Vertreter: **Volmer, Georg, Dipl.-Ing. et al**
**Philips Intellectual Property & Standards GmbH,**
**Postfach 50 04 42**
**52088 Aachen (DE)**

(56) Entgegenhaltungen:
**EP-A- 0 775 923          WO-A-96/03795**
**WO-A-96/05768          WO-A-96/41119**
**WO-A-97/25101          DE-A- 4 215 901**
**FR-E- 93 666          US-A- 5 492 131**

## Beschreibung

Die Erfindung betrifft ein magnetisches Objekt zur Navigation eines in einem von einem Magnetfeld durchsetzten Untersuchungsobjekt. Die Erfindung betrifft außerdem eine Magnet-Resonanz-Anordnung (MR-Anordnung) mit einer Hauptfeldmagnetanordnung zur Erzeugung eines ortsfesten statischen Magnetfeldes und mit einem medizinischen Instrument, insbesondere einem Katheter oder einem flexiblen Endoskop, zur Einführung in ein Untersuchungsobjekt, wobei in oder an dem Instrument ein magnetisches Objekt angeordnet ist.

Ein solches Verfahren ist aus der WO 9603795 A1 bekannt. Dort wird ein passives magnetisches Objekt (ein Permanentmagnet) mittels zeitlich und räumlich veränderlicher Magnetfelder, die von mehreren, insbesondere supraleitenden Spulen erzeugt werden, zu bestimmten Positionen innerhalb des Körpers eines Patienten bewegt. Die Bewegung wird dort mittels Röntgenstrahlung beobachtet.

Eine eingangs genannte MR-Anordnung ist in EP 0 775 923 A2 (PHD 95-125) beschrieben. Dort wird mittels einer an einem Katheter angebrachten Mikrospule die Bewegung des Katheters in einem Untersuchungsobjekt beobachtet, beispielsweise durch Erstellung von MR-Bildern aus von der Mikrospule empfangenen Signalen.

Der Erfindung liegt die Aufgabe zugrunde, ein magnetisches Objekt zur Navigation, das insbesondere für die Magnet-Resonanz-Tomographie geeignet ist, anzugeben. Außerdem liegt der Erfindung die Aufgabe zugrunde, eine zur Navigation eines magnetischen Objektes geeignete MR-Anordnung anzugeben.

Die Aufgabe betreffend das magnetische Objekt wird dadurch gelöst, dass das Objekt ein steuerbares magnetisches Moment aufweist und dass die Bewegungsrichtung des Objektes durch Steuerung des magnetischen Momentes bestimmt wird.

Erfindungsgemäß wird ein aktiver Magnet (ein Elektromagnet) als Objekt verwendet, welcher ein steuerbares magnetisches Moment aufweist, das insbesondere an- und abschaltbar ist. Beim bekannten Verfahren wird dagegen ein passiver Magnet mit unveränderlichem magnetischen Moment verwendet, was für die MR-Tomographie nicht geeignet ist. Zur Steuerung der Bewegungsrichtung des Objektes wird der physikalische Effekt ausgenutzt, dass auf ein Objekt mit einem magnetischen Moment in einem Magnetfeld ein mechanisches Moment wirkt, dessen Richtung und Stärke von der Richtung und der Stärke des magnetischen Momentes und des Magnetfeldes abhängen. Um das Objekt in eine gewünschte Richtung zu lenken, wird erfindungsgemäß ein magnetisches Moment geeigneter Größe und Richtung eingestellt, so dass sich in dem vorhandenen Magnetfeld ein mechanisches Moment in der gewünschten Richtung und mit der erforderlichen Stärke ergibt.

Eine bevorzugte Ausgestaltung der Erfindung ist in Anspruch 2 angegeben. Jede der drei Spulen des Objektes ist einzeln mit einem Strom beaufschlagbar, so dass ein magnetisches Moment in jeder beliebigen Richtung einstellbar ist, wodurch das Objekt in jede Richtung gelenkt werden kann. Die Spulen können gleichzeitig oder nacheinander mit einem Strom beaufschlagt werden.

Die Ausgestaltung der Erfindung gemäß Anspruch 3 ist besonders einfach, platzsparend und leicht zu realisieren. Da bei Verwendung von nur einer oder zwei Spulen nicht in jeder Richtung ein magnetisches Moment eingestellt werden kann, sind bei dieser Ausgestaltung Mittel, insbesondere mechanische Mittel, wie Gelenke und Bowdenzüge, vorgesehen, um die Lage der einzelnen Spulen oder der Spulenanordnung gegenüber dem äußeren Magnetfeld zu verändern und somit auch die Richtung des magnetischen Momentes einer Spule oder der Spulenanordnung zu verändern. Auch hier kann jede Spule einzeln oder alle Spulen gleichzeitig und für eine einstellbare Zeitdauer von einem Strom durchflossen werden, um das gewünschte magnetische Moment einzustellen.

Bevorzugt wird das erfindungsgemäße magnetische Objekt verwendet bei einer MR-Anordnung, wobei dann gemäß Anspruch 5 das Magnetfeld das ortsfeste statische Magnetfeld der MR-Anordnung ist. Dieses Magnetfeld ist insbesondere auch homogen und weist eine hohe Magnetfeldstärke auf, so dass auch mit einer kleinen Spule ein ausreichend großes mechanisches Moment erzeugt werden kann, um das Objekt in dem Magnetfeld ausschließlich mittels des wirkenden mechanischen Momentes zu lenken.

Das erfindungsgemäße magnetische Objekt eignet sich besonders dazu, ein medizinisches Instrument, insbesondere ein Katheter oder ein flexibles Endoskop in einem Untersuchungsobjekt, z.B. im Gefäßsystem des Kopfes eines Patienten, zu bewegen. Dazu ist die Ausgestaltung der Erfindung gemäß Anspruch 6 vorgesehen. Der Vorschub des medizinischen Instruments erfolgt dabei beispielsweise per Hand, während eine Richtungsänderung durch das erfindungsgemäße Verfahren bewirkt wird.

Gemäß der Ausgestaltung des magnetische Objekts nach Anspruch 7 kann auch die Position des Objektes ermittelt werden. Diese Ausgestaltung wird insbesondere bei einer MR-Anordnung und bei einer Ausgestaltung des Objektes als Spulenanordnung verwendet.

Die Aufgabe betreffend die MR-Anordnung wird ausgehend von der eingangs genannten MR-Anordnung dadurch gelöst, dass das Objekt ein steuerbares magnetisches Moment aufweist und dass Mittel vorgesehen sind zur Steuerung des magnetischen Momentes zur Navigation des magnetischen Objektes im Untersuchungsobjekt. Diese Mittel können beispielsweise eine geeignete Steuereinheit und geeignete Stromquellen enthalten, mit denen einzelne Spulen, aus denen das magnetische Objekt vorzugsweise aufgebaut ist, mit Strömen beaufschlagt werden zur Einstellung eines auf das Objekt wirkenden magnetischen Momentes.

Die Erfindung wird nachfolgend anhand der Zeichnungen näher erläutert. Es zeigen:

Fig. 1 ein Blockschaltbild einer erfindungsgemäßen MR-Anordnung,
Fig. 2 ein Katheter mit einer ersten Ausgestaltung einer Spulenanordnung,
Fig. 3 ein Katheter mit einer zweiten Ausgestaltung einer Spulenanordnung und
Fig. 4 ein Katheter mit einer dritten Ausgestaltung einer Spulenanordnung.

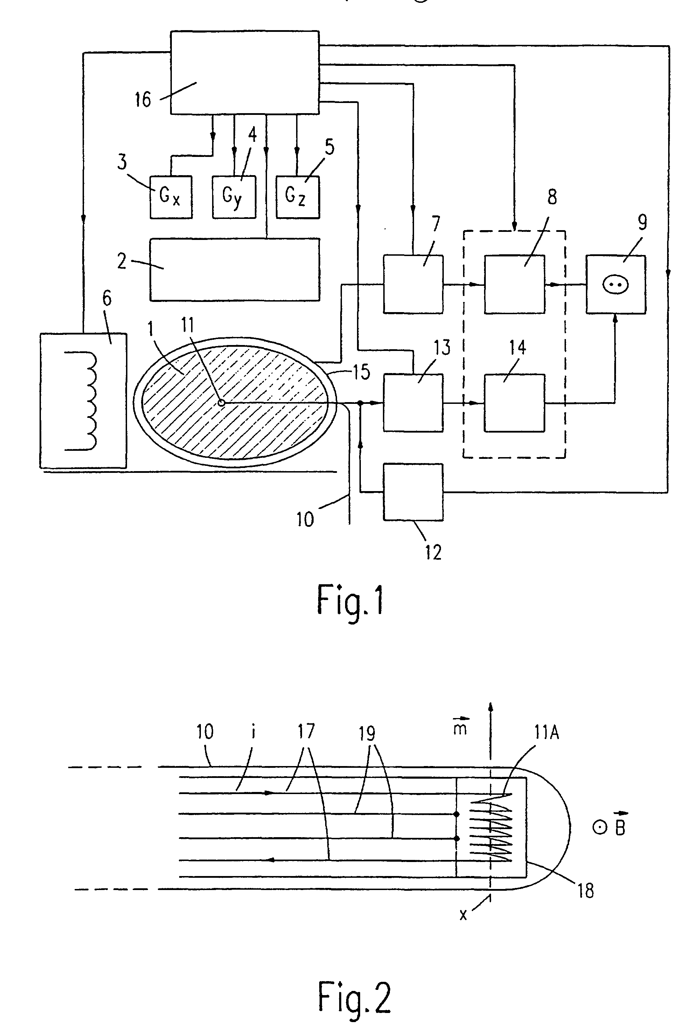

In Fig. 1 ist ein Untersuchungsobjekt 1 in einem Untersuchungsbereich angeordnet, der einem homogenen stationären statischen Magnetfeld ausgesetzt ist, das von einer Hauptfeldmagnetanordnung 2 erzeugt wird. Dem homogenen Magnetfeld können mittels Gradientenspulenanordnungen 3, 4, 5 magnetische Gradientenfelder überlagert werden. Ein Hochfrequenzsender 6 kann im Untersuchungsbereich impulsweise ein hochfrequentes Magnetfeld erzeugen. Im Untersuchungsobjekt 1 erzeugte MR-Signale werden von einer Empfangsspulen-Anordnung 15 in Verbindung mit einer Empfängeranordnung 7 detektiert, und aus den digitalisierten MR-Signalen wird beispielsweise nach einer Fourier-Transformation in einer Rekonstruktionseinheit 8 die Kernmagnetisierungsverteilung im Untersuchungsbereich rekonstruiert und in Form eines MR-Bildes auf einer Wiedergabeeinheit 9 wiedergegeben.

In das Untersuchungsobjekt 1 ist ein medizinisches Instrument eingeführt, beispielsweise ein Katheter 10, an dessen Spitze eine Spule 11 geringen Durchmessers (eine Mikrospule) befestigt ist. Die von der Spule 11 empfangenen Signale werden einer Empfangeranordnung 13 zugeführt, digitalisiert und anschließend einer Auswerteeinheit 14 zugeführt, die die Position der Spule 11 ermittelt und in das auf der Wiedergabeeinheit 9 dargestellte MR-Bild einblendet. Wie gestrichelt angedeutet ist, können die Komponenten 8 und 14 mittels eines geeignet programmierten Rechners realisiert werden. Die Komponenten 2 bis 15 werden durch eine programmierbare Steuereinheit 16 gesteuert.

Eine Spulen-Steuereinheit 12 steuert die Einstellung des magnetischen Momentes der Spule 11, wenn diese nicht auf Empfang geschaltet ist. Die Spulen-Steuereinheit 12 weist dazu eine Stromquelle auf, mittels der die Spule 11 mit einem Gleichstrom beaufschlagt werden kann, um ein magnetisches Moment zu erzeugen.

Ein Katheter 10 mit einer einzelnen zylindrischen, aus mehreren Windungen bestehenden Spule 11A ist vergrößert in Fig. 2 gezeigt. Über Stromleitungen 17 kann der Spule 11A ein Strom i zugeführt werden, wodurch ein magnetisches Moment $\vec{m}$ parallel zur

Längsachse x entsteht: $\vec{m} = i \cdot N \cdot \vec{S}$ , wobei i der durch die Spule fließende Strom, N die Anzahl der Windungen und $\vec{S}$ der Vektor auf die Querschnittsfläche der Spule mit dem Radius r ($S = r^2 \cdot \pi$) ist. In einem statischen Magnetfeld $\vec{B}$ in der gezeigten Richtung senkrecht zu $\vec{m}$ entsteht das mechanische Moment $\vec{M} = m \times \vec{B}$ , dessen Betrag maximal ist, wenn das Magnetfeld $\vec{B}$ senkrecht auf dem magnetischen Moment $\vec{m}$ steht, und den Wert

$$\max(|\vec{M}|) = \pi \cdot i \cdot N \cdot r^2 \cdot |\vec{B}| \text{ hat.}$$

Die maximale Kraft am Rand der Spule 11A beträgt etwa:

$$\max(F) = \max(|\vec{M}|) / r .$$

Das mechanische Moment bewirkt also, daß das Katheter 10 mit der Spule 11A gedreht wird um eine senkrecht auf dem Magnetfeld $\vec{B}$ und senkrecht auf dem magnetischen Moment $\vec{m}$ stehende, also in Richtung des mechanischen Moments $\vec{M}$ verlaufende Achse.

Bei einer Flußdichte von 1,5T, einem Strom von 1A und einer Spule von 1mm Radius und zehn Windungen ergibt sich eine maximale Kraft von 0,02N, die bei einem entsprechend biegsamen Kathetermaterial für die Lenkung des Katheters ausreichend ist.

Die Spule 11A ist bei der Ausführung gemäß Fig. 2 in einer Hülle 18 angeordnet, an der mechanische Zugdrähte 19 angebracht sind zur Veränderung der Lage der Spule bezüglich der Richtung des Magnetfeldes $\vec{B}$ . Bei Veränderung der Lage der Spule 11A wird auch die Richtung des magnetischen Momentes $\vec{m}$ verändert, so daß auch das resultierende mechanische Moment eine andere Richtung aufweist und sich eine Kraftwirkung auf die Spitze des Katheters 10 in eine andere Richtung ergibt.

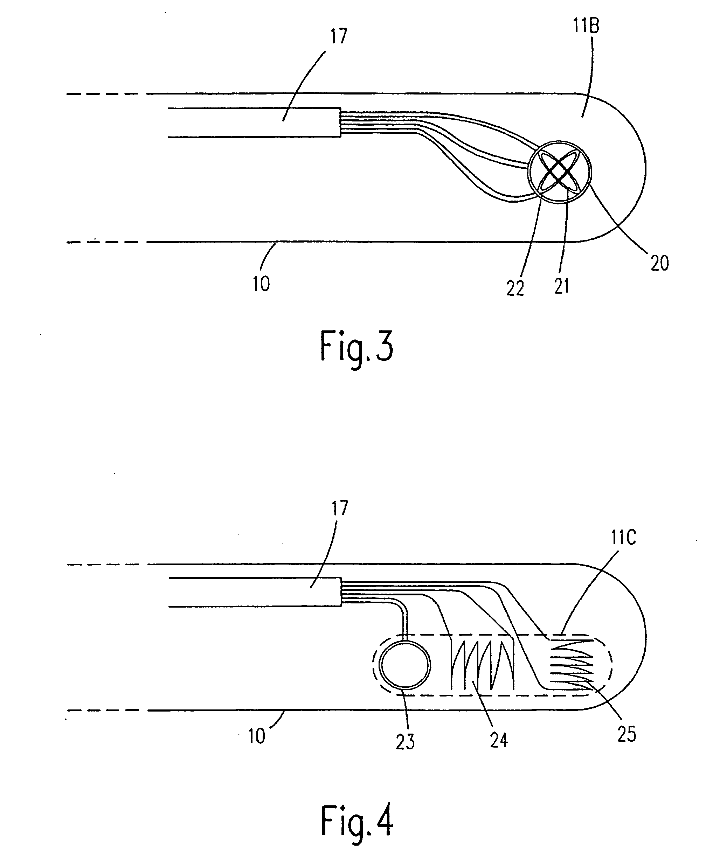

Eine alternative Ausgestaltung eines Katheters 10 ist in Fig. 3 gezeigt. Die Spulenanordnung 11B besteht dabei aus drei um einen gemeinsamen Mittelpunkt, jeweils senkrecht zueinander angeordneten Spulen 20, 21, 22, die jeweils aus mehreren Windungen bestehen können. Über eine Zuleitung 17 ist jede der drei Spulen 20, 21, 22 einzeln mit einem Strom beaufschlagbar, um ein magnetisches Moment einzustellen, so daß sich in dem äußeren Magnetfeld ein mechanisches Moment in der gewünschten Richtung ergibt. Die Spulen 20, 21, 22 können dabei gleichzeitig oder zeitlich nacheinander mit einem Strom der benötigten Größe und für die benötigte Zeitdauer beaufschlagt werden.

Eine weitere alternative Ausgestaltung zeigt Fig. 4, bei der die Spulenanordnung 11C ebenfalls aus drei zueinander senkrecht angeordneten Spulen 23, 24, 25 ausgestaltet ist, die jedoch in dem Katheter 10 hintereinander angeordnet sind.

Statt der gezeigten ringförmigen Solenoidspulen können auch Sattelspulen verwendet werden, die auf einem zylindrischen Körper an der Spitze des Katheters angeordnet sind. Zur Veränderung der Lage einer Spule oder der Spitze des Katheters können statt der genannten mechanischen Zugdrähte auch Materialien verwendet werden, die eine bestimmte Lage oder Krümmung annehmen, sobald eine Gegenkraft entfällt. Auch die Verwendung spezieller Katheter (z.B. Pigtail-Katheter), bei denen ein weicher, gekrümmter Draht aus einer relativ starren Hülle vorgeschoben wird und sich so in eine bestimmte Lage oder Richtung bringen läßt, sind denkbar.

Die Spule 11 (siehe Fig. 1) muß nicht notwendigerweise sowohl Signale empfangen können (zur Bestimmung der Lage der Spule) als auch zur Bewegung des Katheters im Untersuchungsobjekt geeignet sein. Es können auch mehrere Spulen für getrennte Zwecke vorgesehen sein.

Die Erfindung wird insbesondere verwendet zur Navigation eines Katheters im Gefäßsystem des Kopfes, beispielsweise um in einem Gefäß eine Blutgerinsel aufzulösen. Dazu muß das Katheter aus einem Hauptgefäß in die richtigen Nebengefäße eingeführt werden, wozu ein lokales Krümmen der Katheterspitze erforderlich ist. Eine Darstellung des Gefäßsystems kann beispielsweise mittels eines MR-Angiographie-Verfahrens vorab erhalten werden.

## Patentansprüche

1. Magnetisches Objekt (11) zur Navigation in einem von einem Magnetfeld durchsetzten Untersuchungsobjekt (1),
   **dadurch gekennzeichnet, dass** das Objekt (11) ein steuerbares magnetisches Moment aufweist und dass die Bewegungsrichtung des Objektes (11) durch Steuerung des magnetischen Momentes bestimmt werden kann.

2. Magnetisches Objekt nach Anspruch 1,
   **dadurch gekennzeichnet, dass** das Objekt (11) eine durch drei zueinander orthogonal angeordnete Spulen (20, 21, 22; 23, 24, 25) gebildete Spulenanordnung (11 B, 11 C) aufweist.

3. Magnetisches Objekt nach Anspruch 1,
   **dadurch gekennzeichnet, dass** das Objekt (11) eine durch eine oder zwei zueinander orthogonal angeordnete Spulen gebildete Spulenanordnung (11A) vorgesehen ist und dass Mittel (18, 19) zur Veränderung der Lage der Spulenanordnung (11A) gegenüber dem Magnetfeld vorgesehen sind.

4. Magnetisches Objekt nach Anspruch 2 oder 3,
   **dadurch gekennzeichnet, dass** zur Steuerung der Bewegungsrichtung des Objektes (11) die Spulen (11A; 20, 21, 22; 23, 24, 25) zeitweise von Strömen durchflossen werden und dass Mittel (12) zur Speisung der Spulen (11A; 20, 21, 22; 23, 24, 25) mit Strömen vorgesehen sind.

5. Magnetisches Objekt nach Anspruch 1,
   **dadurch gekennzeichnet, dass** das Magnetfeld das ortsfeste statische Magnetfeld einer Magnet-Resonanz-Anordnung ist.

6. Magnetisches Objekt nach Anspruch 1,
   **dadurch gekennzeichnet, dass** das Objekt (11) in oder an einem medizinischen Instrument (10), insbesondere einem Katheter, angeordnet ist.

7. Magnetisches Objekt nach Anspruch 1,
   **dadurch gekennzeichnet, dass** das Objekt (11) zum Erzeugen und/oder Detektieren eines magnetischen Feldes ausgestaltet ist und dass die Position des Objektes (11) mit geeigneten Mitteln (13, 14) anhand des von dem Objekt (11) erzeugten oder detektierten Magnetfeldes bestimmt wird.

8. MR-Anordnung mit einer Hauptfeldmagnetanordnung (2) zur Erzeugung eines ortsfesten statischen Magnetfeldes und mit einem medizischen Instrument (10), insbesondere einem Katheter oder einem flexiblen Endoskop, zur Einführung in ein Untersuchungsobjekt (1), wobei in oder an dem Instrument (10) ein magnetisches Objekt (11) angeordnet ist,
   **dadurch gekennzeichnet, dass** das Objekt (11) ein steuerbares magnetisches Moment aufweist und dass Mittel (12) vorgesehen sind zur Steuerung des magnetischen Momentes zur Navigation des magnetischen Objektes (11) im Untersuchungsobjekt (1).

9. Medizinisches Instrument, **dadurch gekennzeichnet, dass** in oder an ihm ein magnetisches Objekt nach Anspruch 1 angebracht ist.

## Claims

1. A magnetic object (11) for navigating in an object (1) to be examined which is permeated by a magnetic field,
   **characterized in that** the object (11) has a controllable magnetic moment and that the direction of movement of the object (11) can be determined by control of the magnetic moment.

2. A magnetic object as claimed in Claim 1,
   **characterized in that** the object (11) is formed by a coil system (11B, 11C) which consists of three mutually orthogonal coils (20, 21, 22; 23, 24, 25).

3. A magnetic object as claimed in Claim 1,

**characterized in that** the object (11) is formed by a coil system (11A) which consists of one or two mutually orthogonal coils, and that means (18, 19) are provided for changing the position of the coil system (11A) relative to the magnetic field.

4. A magnetic object as claimed in Claim 2 or 3, **characterized in that** currents are temporarily applied to the coils (11A; 20, 21, 22; 23, 24, 25) in order to control the direction of movement of the object (11), and that means (12) are provided for supplying the coils (11A; 20, 21, 22; 23, 24, 25) with currents.

5. A magnetic object as claimed in Claim 1, **characterized in that** the magnetic field is the stationary, static magnetic field of a magnetic resonance device.

6. A magnetic object as claimed in Claim 1, **characterized in that** the object (11) is arranged in or on a medical instrument (10), notably a catheter.

7. A magnetic object as claimed in Claim 1, **characterized in that** the object (11) is arranged to generate and/or detect a magnetic field and that the position of the object (11) is determined by means of suitable means (13, 14) utilizing the magnetic field generated or detected by the object (11).

8. An MR device which includes a main field magnetic system (2) for generating a stationary, static magnetic field and a medical instrument (10), notably a catheter or a flexible endoscope, which is to be introduced into an object (1) to be examined, a magnetic object (11) being provided in or on the instrument (10), **characterized in that** the object (11) has a controllable magnetic moment and that there are provided means (12) for controlling the magnetic moment for navigating the magnetic object (11) within the object (1) to be examined.

9. A medical instrument, **characterized in that** a magnetic object as claimed in Claim 1 is arranged in or on it.

## Revendications

1. Objet magnétique (11) pour la navigation dans un objet à examiner traversé par un champ magnétique (1), **caractérisé en ce que** l'objet (11) présente un moment magnétique commandable et que la direction du mouvement de l'objet (11) peut être déterminée par commande du moment magnétique.

2. Objet magnétique selon la revendication 1, **caractérisé en ce que** l'objet (11) présente un montage de bobines (11B, 11C) formé par trois bobines (20, 21, 22; 23, 24, 25) disposées orthogonalement l'une par rapport à l'autre.

3. Objet magnétique selon la revendication 1, **caractérisé en ce que** l'objet (11) présente un montage de bobines (11A) formé par une ou deux bobines disposées orthogonalement l'une par rapport à l'autre et que des moyens (18, 19) sont prévus pour le changement de position du montage de bobines (11A) par rapport au champ magnétique.

4. Objet magnétique selon l'une des revendications 2 ou 3, **caractérisé en ce que** les bobines (11A; 20, 21, 22; 23, 24, 25) sont traversées temporairement par des courants pour la commande de la direction de mouvement de l'objet (11) et que des moyens (12) sont prévus pour l'alimentation des bobines (11A; 20, 21, 22; 23, 24, 25) avec des courants.

5. Objet magnétique selon la revendication 1, **caractérisé en ce que** le champ magnétique est le champ magnétique statique stationnaire d'un dispositif à résonance magnétique.

6. Objet magnétique selon la revendication 1, **caractérisé en ce que** l'objet (11) est disposé dans ou sur un instrument médical (10), en particulier un cathéter.

7. Objet magnétique selon la revendication 1, **caractérisé en ce que** l'objet (11) est conçu pour la production et/ou la détection d'un champ magnétique et que la position de l'objet (11) est déterminée avec des moyens adéquats (13, 14) à l'aide du champ magnétique détecté ou produit par l'objet (11).

8. Montage RM avec un champ magnétique principal (2) pour la production d'un champ magnétique statique stationnaire et avec un instrument médical (10), en particulier un cathéter ou un endoscope flexible, à introduire dans un objet à examiner (1), un objet magnétique (11) étant disposé dans ou sur l'instrument (10), **caractérisé en ce que** l'objet (11) présente un moment magnétique à commander et que des moyens (12) sont prévus pour commander le moment magnétique en vue de la navigation de l'objet magnétique (11) dans l'objet à examiner (1).

9. Instrument médical, **caractérisé en ce qu'**un objet magnétique, selon la revendication 1, est placé dans ou sur lui.

Fig.1

Fig.2

Fig.3

Fig.4